# EUROPEAN PATENT APPLICATION

(11) **EP 1 159 959 A1**
(43) Date of publication of application: **05.12.2001**
(21) Application number: 98961478.9
(22) Date of filing: 22.12.1998
(51) Int. Cl.: A61K 31/195

(54) **PREVENTIVE FOR RESPIRATORY INFECTIOUS DISEASES**

(71) Applicant: KYORIN PHARMACEUTICAL CO., LTD., Chiyoda-ku, Tokyo 101-0062 (JP); Nagatake, Tsuyoshi, Nagasaki-shi, Nagasaki 852-8523 (JP)
(72) Inventor: NAGATAKE, Tsuyoshi, Institute of Tropical Medicine, Nagasaki-shi, Nagasaki 852-8523 (JP)
(74) Representative: VOSSIUS & PARTNER
(86) International application number: JP9805810
(87) International publication number: WO0037070

(57) **Abstract**

A preventive against respiratory infectious disease is disclosed which contains carbocysteine represented by Chemical Formula (1) as the active ingredient: 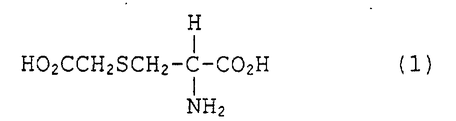 This preventive of the present invention can be a preventive against the respiratory infectious disease, effective in the initial-infective stage, namely effective to suppress adhesion of the bacteria onto an upper respiratory tract. The carbocysteine is promising in decrease of acute exacerbation frequency, and prevention of bacterial infection of humans having depressed immunity, and retardation of the increase of tolerant bacteria caused by wide use of antimicrobials.

## Description

### Background of the Invention:

### Field of the Invention:

The present invention relates to a preventive against a respiratory infectious disease which prevents adhesion of contagium onto the upper respiratory tract.

### Description of the Related Art:

For treatment of a respiratory infectious disease, after-treatments against the infection have been widely investigated, such as bactericidal disinfection by an antimicrobial, anti-inflammation treatment by antiinflammatory agent, promotion of excretion of sputum by an expectorant, and so forth. However, for prevention of the infection, only virus adhesion-inhibiting agents are being developed although the prevention of infection is recognized to be important. The prevention of adhesion of the bacterial cells onto the respiratory tract has not been reported at all. No effective medicine is known for this purpose.

The adhesion ability of Moraxella (Branhamela) catarrhalis, which is known as one of the five kinds of main inflammation-causing bacteria, onto pharynx epithelium (mucous membrane of upper respiratory tract) is reported to correlate significantly with development of the infection in the lower respiratory tract (Mubaki N. et al.: Tohuku J. Exp. Med. 153, 111-121, 1987).

From this, it is expected that prevention of the adhesion of the respiratory contagium onto the upper respiratory tract could be the first step of preventing the lower respiratory tract infection. Actually, mouth-washing with a disinfectant has been proved clinically to be effective for the prevention of the infection. However, no medicine has been reported which prevents directly the adhesion of the respiratory contagium onto the upper respiratory tract.

The wide use of the antimicrobials against the respiratory infectious disease has produced various new problems such as increase of tolerable bacteria due to the fact that infants and elders often suffer from repeated infection by virus, bacteria, etc. Patients with chronic respiratory infectious disease or with immune depression against various disease germs are facing the danger of the repeated infection with the bacteria. The establishment of an effective infection prevention method for such patients is the problem to be solved. Therefore, it is desirable for such easily infectious patients to prevent the disease before the human body is infected by a respiratory contagium.

### Disclosure of the Invention:

The inventors of the present invention noticed that the first step of the respiratory infectious disease is adhesion of the contagium onto an upper respiratory tract, and expected that the development of the respiratory disease could be prevented by inhibiting adherence of the contagium onto the respiratory tract. Therefore, the inventors of the present invention tested carbocysteine, a well-known expectorant, for the effect of prevention of adhesion of a bacteria onto the respiratory tract, and found the remarkable effect thereof.

The present invention relates to a preventive against a respiratory infectious disease, the preventive containing, as the active ingredient, carbocysteine represented by the chemical formula (1) below:

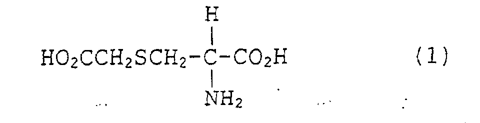

The carbocysteine is a cysteine derivative represented by the chemical formula (1), being useful as an expectorant. The carbocysteine was developed by Laboratories Joulie Co. in France, and was commercialized in 1965 with the trade name "Rhinathiol". Later in United Kingdom, carbocysteine was commercialized by Berk Pharmaceuticals Co. with a trade name "Mucodyne" in 1972. At present, carbocysteine is commercialized in 14 countries in the world.

In Japan, carbocysteine was developed by Kyorin Pharmaceutical Co., Ltd., approved for production by Ministry of Health and Welfare (Japan) in 1981, and commercialized with trade name "Mucodyne", and is being used widely as a safe expectorant.

Carbocysteine is known to have various effects: promotion of excretion of sputum by improving its properties (Brown D.T: Drug Intelligence Clin. Pharmacol., 22. 603-608, 1988), promotion of repair of the cilium and improvement of its transporting ability (Ogihara M. et al.: Kikanshigaku (Treatise on Bronchia), 1982), and so forth. However, its effect of inhibiting adhesion of bacteria has not been known.

Carbocysteine as a respiratory infectious disease preventive can be administered to a human body in a pharmaceutically known formulation form through a known administration route. For example, carbocysteine can be administered orally in a form of such as powders, tablets, capsules, grains, granules, and syrups. The amount of administration of carbocysteine as the preventive against the respiratory infectious disease ranges preferably from 250 to 2000 mg, more preferably from 250 to 1000 mg per dose, and preferably three doses per day, depending on the age, the body weight, and the symptom of the patient.

### Brief Description of the Drawings:

Fig. 1 is a graph showing the test results of Example 1.

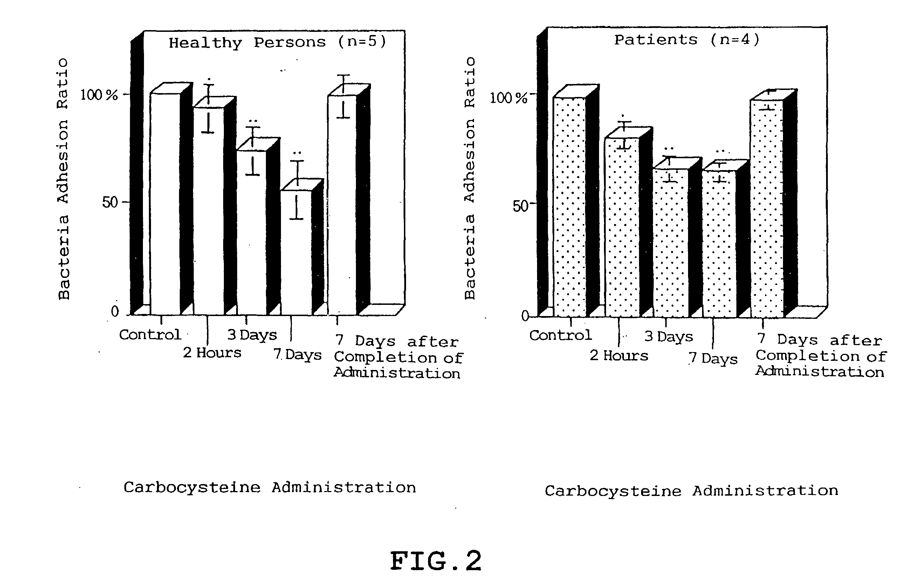

Fig. 2 is a graph showing the test results of Example 2.

### Examples:

### [Example 1]

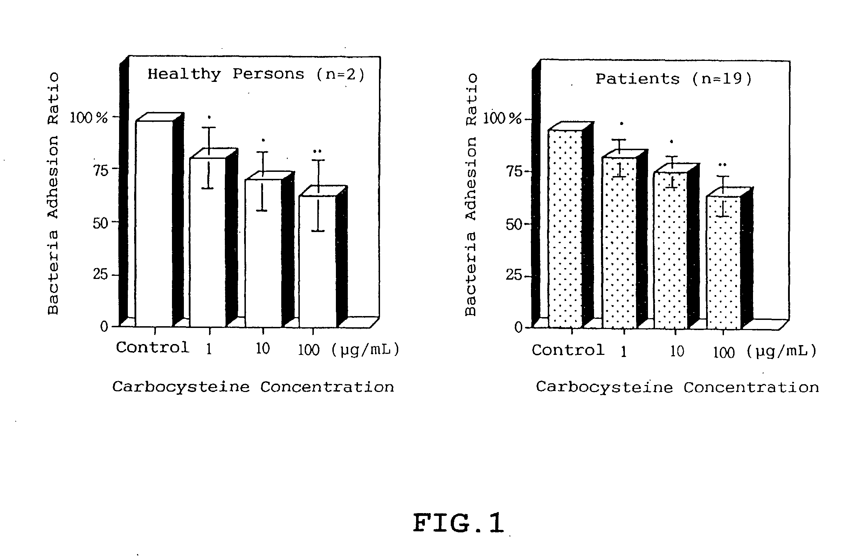

The effect of the carbocysteine for inhibiting the adhesion of Moraxella (Branhamela) catarrhalis, a respiratory contagium, onto a human pharynx epithelium cells was evaluated by vitro experiments.
(1) Pharynx epithelium cells:
   With full informed consent, cell samples were collected by rubbing, with swabs, portions of pharynx of two healthy persons of 28-54 years of age and of 19 patients of 53-75 years of age having respiratory infectious disease.
(2) Moraxella (Branhamela) catarrhalis:
   The bacterial cells were isolated from the expectoration, and the isolated cells were cultured, by using clinically separated strains having clear inflammation tendency.
(3) Adhesion test:
   A liquid suspension of Moraxella (Branhamela) catarrhalis was mixed with a liquid suspension of the pharynx epithelium cells. A solution of carbocysteine was added thereto to attain the final concentrations ranging from 1 to 100 µg/mL. After left standing for a prescribed time, the Moraxella (Branhamela) catarrhalis not adhering to the pharynx epithelium cells were removed by centrifugation. The remaining pharynx epithelium cells were fixed with a cyto-spin onto a slide glass, and were stained by Gram's method. The number of Moraxella (Branhamela) catarrhalis adhering on the pharynx epithelium was counted with an optical microscope. A control was employed which did not contain carbocysteine.
(4) Evaluation:
   In the microscopical examination, fifty cells of the pharynx epithelium were randomly selected. The average number of Moraxella (Branhamela) catarrhalis adhering on one pharynx epithelium cell randomly selected was counted. The adhesion ratio was calculated in comparison with the control taken as 100%.
(5) Results:
   Carbocysteine inhibited the adhesion of Moraxella (Branhamela) catarrhalis to the pharynx epithelium cells of the healthy persons and patients as shown in Fig. 1.

### [Example 2]

The effect of the carbocysteine for inhibiting the adhesion of Moraxella (Branhamela) catarrhalis onto a human pharynx epithelium cells was evaluated by oral administration.
(1) Objects:
   Five healthy persons of 30-54 years of age, and four patients of 50-75 years of age having chronic obstructive pulmonary disease were selected as the objects with full informed consent.
(2) Administration method:
   Carbocysteine was administered orally at a dose of 500 mg, three times a day for 7 days.
(3) Adhesion test:
   The pharynx epithelium cells were collected in the same manner as in Example 1 before the carbocysteine administration; 2 hours after the first administration; 3 days and 7 days after the start of the administration; and 7 days after completion of the administration. The respective liquid suspensions of the pharynx epithelium cells were mixed with a liquid suspension of Moraxella (Branhamela) catarrhalis. After a prescribed time, the Moraxella (Branhamela) catarrhalis not adhering to the pharynx epithelium cells were washed and removed by centrifugation. The remaining pharynx epithelium cells were fixed with a cyto-spin onto a slide glass, and were stained by Gram's method. The number of strains of Moraxella (Branhamela) catarrhalis adhering on the pharynx epithelium cells was counted with an optical microscope. The pharynx epithelium cells collected before the carbocysteine administration were used as the control.
(4) Evaluation:
   In the microscopical examination, fifty cells were randomly selected. The average number of Moraxella (Branhamela) catarrhalis adhering per one of the fifty pharynx epithelium cells randomly selected was counted. The adhesion ratio was calculated in comparison with the control taken as 100%.
(5) Results:
   The adhesion ratio was lowered by administration of carbocysteine. The adhesion ratio decreases during the continued administration with lapse of time, and became. lowest by 7 days of the administration. After completion of the administration, the ratio increased to the level near 100 % in 7 days. Table 2 shows the results.

### Industrial Applicability:

The inventors of the present invention have found that carbocysteine inhibits effectively the adhesion of respiratory contagium onto pharynx epithelium cells in vitro as well as in vivo (oral administration). Therefore, carbocysteine can be a preventive against the respiratory infectious disease, being effective in the initial-infective step, namely effective to suppress adhesion of the bacteria onto an upper respiratory tract. The carbocysteine is promising in decrease of acute exacerbation frequency, and prevention of bacterial infection of humans having depressed immunity, and retardation of the increase of tolerant bacteria caused by wide use of the antimicrobials.

## Claims

1. Use of 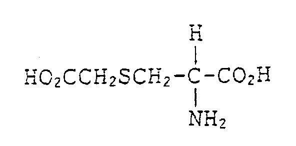 for the preparation of a preventive agent against infectious disease.
